(19) Europäisches Patentamt
European Patent Office
Office européen des brevets

(11) Veröffentlichungsnummer: **0 193 736**
**B1**

# (12) EUROPÄISCHE PATENTSCHRIFT

(45) Veröffentlichungstag der Patentschrift: 05.07.89

(51) Int. Cl.⁴: C 08 B 37/08, A 61 K 7/48

(21) Anmeldenummer: 86101241.7

(22) Anmeldetag: 30.01.86

## ERRATUM

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE, COLONNE, LIGNE) | | | LAUTET BERICHTIGT:<br>SHOULD READ :<br>DEVRAIT ETRE LU : |
|---|---|---|---|---|
| $HO\left[C_6H_{11-m-q}NO_4(R^1)_m(TR^2)_n(R^3)_q\right]_pH$ (I), | 10 | 17 | 11/13 | $HO\left[C_6H_{11-m-q}NO_4(R^1)_m(\ R^2)_n(R^3)_q\right]_pH$ (I), |
| $\left[-CH_2-\underset{CH_3}{CH}-O-\ -R-^{a},\right]_x$ | 10 | 18 | 14/18 | $\left[-CH_2-\underset{CH_3}{CH}-O-\right]_x-Ra$ |
| 50 of 96% to acetylated | 13 | 24 | 1 | 50 to 96% to deacetylated |

Tag der Entscheidung über die Berichtigung / Date of decision on rectification / Date de décision portant sur modification: 07.08.89

Ausgabe- und Veröffentlichungstag / Issue and publication date / Date d'edition et de publication: 18.10.89

Patbl.Nr / EPB no / Bull. no: 89/42

(19) Europäisches Patentamt
European Patent Office
Office européen des brevets

(11) Veröffentlichungsnummer: **0 193 736**
**B1**

(12) # EUROPÄISCHE PATENTSCHRIFT

(45) Veröffentlichungstag der Patentschrift: **05.07.89**

(51) Int. Cl.⁴: **C 08 B 37/08**, A 61 K 7/48

(21) Anmeldenummer: **86101241.7**

(22) Anmeldetag: **30.01.86**

(54) **Kosmetische Mittel auf der Basis von alkyl-hydroxypropyl-substituierten Chitosanderivaten, neue Chitosanderivate sowie Verfahren zu ihrer Herstellung.**

(30) Priorität: **07.02.85 DE 3504095**

(43) Veröffentlichungstag der Anmeldung: **10.09.86 Patentblatt 86/37**

(45) Bekanntmachung des Hinweises auf die Patenterteilung: **05.07.89 Patentblatt 89/27**

(84) Benannte Vertragsstaaten: **AT BE CH DE FR GB IT LI LU NL SE**

(56) Entgegenhaltungen:
**EP-A- 0 028 126**
**EP-A- 0 097 229**

**CHEMICAL ABSTRACTS, Band 98, Nr. 22, Mai 1983, Seite 105, Nr. 181486e, Columbus, Ohio, US; & JP - A - 57 180 602 (NIPPON SODA CO., LTD.) 06-11-1982**
**BULLETIN OF THE CHEMICAL SOCIETY OF JAPAN, Band 26, Nr. 3, 1953, Seiten 148-153, Chemical Society of Japan, Tokyo, JP; R. SENZYU: "Untersuchungen über Lignin und Zellstoff. II. Eine neue Bestimmungsmethode des Lignins in Hölzern und Zellstoffen durch Kolloidtitration"**

(73) Patentinhaber: **Wella Aktiengesellschaft, Berliner Allee 65, D-6100 Darmstadt (DE)**

(72) Erfinder: **Lang, Günther, Dr., Auf der Roten Erde 10 B, D-6107 Reinheim 5 (DE)**
Erfinder: **Maresch, Gerhard, Hölgesstrasse 19, D-6100 Darmstadt (DE)**
Erfinder: **Lenz, Hans-Rudi, Motkestrasse 16, D-6100 Darmstadt (DE)**
Erfinder: **Konrad, Eugen, Mecklenburger Strasse 101, D-6100 Darmstadt (DE)**
Erfinder: **Breuer, Lothar, Montmeyrauer Strasse 19, D-6101 Gross-Bieberau (DE)**
Erfinder: **Hoch, Dietrich, Ringstrasse 48, D-6102 Pfungstadt (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft kosmetische Mittel zur Behandlung von Haaren, insbesondere Mittel zur Festigung der Frisur, oder der Haut mit einem Gehalt an neuen makromolekularen, von Chitosan abgeleiteten Verbindungen in einer geeigneten Kosmetikgrundlage.

Die Erfindung betrifft weiterhin die neuen Chitosanderivate sowie Verfahren zu ihrer Herstellung.

Die heute auf dem Markt befindlichen Haar- und Frisurenfestiger enthalten üblicherweise synthetische, gegebenenfalls auswaschbare Kunstharze, wie z.B. Polyvinylpyrrolidon, Mischpolymerisate von Vinylpyrrolidon mit Vinylacetat, Terpolymerisate von Vinylacetat mit anderen Monomeren, Polyacrylate und Polymethacrylate und deren Copolymere mit anderen Monomeren.

Die genannten Polymeren weisen bei ihrem Einsatz in Haarspray-Rezepturen verschiedene Nachteile auf. So verbleiben häufig die Lösungsmittel sehr lange in dem auf dem Haar aufgebrachten Harzfilm, so dass eine relativ lange Trocknungszeit notwendig ist, und das Haar fühlt sich direkt nach dem Aufsprühen des Haarsprays klebrig an.

Weiterhin führt der Einsatz der meisten dieser Polymere zu einer starken Vernetzung der Oberfläche der Frisur (sogenannte Helmbildung), was dem Haar ein unnatürliches Aussehen und einen rauhen Griff verleiht.

Ferner ist mit den meisten der genannten Polymere die Auskämmbarkeit des Haares nach der Anwendung stark verschlechtert, und das Frisieren wird wegen einer zu hohen statischen Aufladung sehr schwierig.

In letzter Zeit werden aus ökologischen Gründen halogenhaltige Treibgase immer mehr durch halogenfreie ersetzt. Es wurde jedoch festgestellt, dass sich hierzu die Mehrzahl der genannten Polymere nicht eignet. So kann es zu Ausfällungen der Polymere während der Lagerung kommen, wodurch die Sprays unbrauchbar werden.

Man hat bereits versucht, diese vorerwähnten Nachteile dadurch zu beheben, dass wasserlösliche Salze des Chitosans, eines durch Entacetylierung von Chitin herstellbaren Polyglucosamins, in derartigen kosmetischen Mitteln Anwendung finden. In diesem Zusammenhang wird Bezug genommen auf die eigenen Patentschriften EP-PS 0 002 506 und DE-PS 2 627 419.

Das Chitosan ist jedoch in wasserfreien Präparaten, wie insbesondere in wasserfreien Aerosolhaarsprays auf der Grundlage von organischen Lösungsmitteln, unlöslich und kann daher dort nicht verwendet werden. Ein weiterer Nachteil des Chitosans besteht darin, dass es in neutraler oder alkalischer wässriger Lösung unlöslich ist, so dass seine Anwendung beispielsweise in alkalischen Dauerwellmitteln oder Haarfärbemitteln nicht möglich ist.

In organischen Lösungsmitteln lösliche Chitosanderivate werden in der Literatur, bis auf wenige Ausnahmen (siehe z.B. Fujii et al., Carbohydr. Res. 83, Seite 389–393 (1980)), nicht angeführt.

Es wurde nunmehr gefunden, dass sich Chitosan auf einfache Weise durch zwei nacheinander oder zugleich durchgeführte Alkylierungsschritte in Mischätherderivate mit interessanten Eigenschaften umwandeln lässt.

Die hierzu notwendige N-Hydroxypropylierung und die sich anschliessende O-Alkylierung führen zu modifizierten, natürlichen Polymeren, die sich neben der Wasserlöslichkeit im sauren pH-Bereich insbesondere durch ihre gute Löslichkeit in organischen Lösungsmitteln auszeichnen. Gegenstand der Erfindung sind dementsprechend auf diese Weise erhältliche neue makromolekulare, vom Chitosan abgeleitete polymere Verbindungen der allgemeinen Formel I

$$HO\,[C_6H_{11\text{-}m\text{-}q}NO_4(R^1)_m(R^2)_n(R^3)_q]_p H \qquad (I),$$

wobei m jeden beliebigen Zahlenwert von 0 bis 0,6 bedeutet, n jeden beliebigen Zahlenwert von 0,5 bis 10 bedeutet, q ein beliebiger Zahlenwert von 0,5 bis 4,0 ist, p eine ganze Zahl von 50 bis 5000 bedeutet, $R^1$ ein Acetyl-Rest ist, $R^2$ einen zweiwertigen Rest

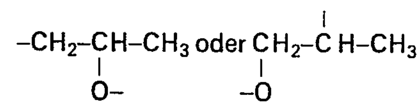

darstellt und $R^3$ einen geradkettigen oder verzweigten Alkylrest mit 1 bis 6 Kohlenstoffatomen bedeutet, oder deren Salze mit einer organischen oder anorganischen Säure.

Der Ausdruck in der eckigen Klammer der Formel I soll dabei sich wiederholende substituierte Glucosamin-Monomereinheiten darstellen.

In der Formel I sollen die Symbole n und q vorzugsweise die Bedeutungen n = 1 bis 3 und q = 1 bis 3,6 haben, während die Grenzviskositätszahl der Chitosanderivate bevorzugt im Bereich 14 bis 44 ml/g liegt.

Von den durch die vorstehende allgemeine Formel definierten Chitosanderivaten sollen besonders bevorzugt sein Verbindungen aufgebaut aus

4 bis 50 Mol-% Einheiten der allgemeinen Formel

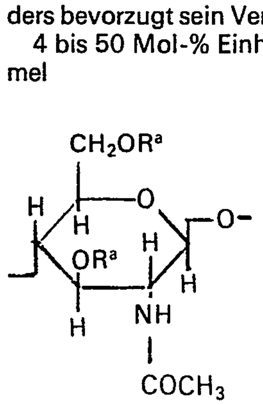

(A)

40 bis 96 Mol-% Einheiten der allgemeinen Formel

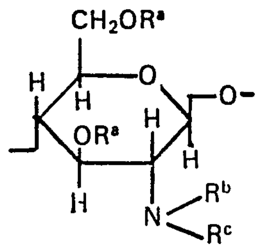

(B) und

0 bis 10 Mol-% Einheiten der allgemeinen Formel

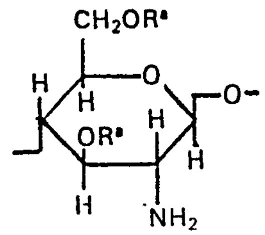

(C)

in statistischer Verteilung, wobei $R^a$ die Bedeutung H oder $C_1$–$C_6$-Alkyl hat, $R^b$ einen Rest H oder $\left[-CH_2-\underset{CH_3}{\underset{|}{C}}H-O-\right]_x-R^a$, mit x = eine ganze Zahl von 1 bis 5, darstellt und $R^c$ die Bedeutung $\left[-CH_2-\underset{CH_3}{\underset{|}{C}}H-O-\right]_x-R^a$ hat, unter der Voraussetzung, dass mindestens einer der Substituenten $R^a$ in den Formeln A oder B $C_1$–$C_6$-Alkyl bedeutet.

Die alkyl-hydroxypropylsubstituierten Chitosanderivate der Formel I werden erfindungsgemäss erhalten, indem man Chitosan entweder in einer 2-Stufenreaktion zunächst mit Propylenoxid und dann mit einem Alkylhalogenid umsetzt oder beide Alkylierungsmittel gleichzeitig, jedoch bei zwei verschiedenen Reaktionstemperaturen, einwirken lässt.

Das bevorzugte Verfahren zur Herstellung der Verbindungen der allgemeinen Formel I ist dadurch gekennzeichnet, dass man ein Chitosan, bestehend aus zu 50 bis 96% entacetyliertem Chitin, in einer 1. Reaktionsstufe in Gegenwart eines organischen Dispergiermittels bei einer Temperatur von 20 °C bis 120 °C, vorzugsweise 80°–100 °C, mit Propylenoxid umsetzt und das erhaltene Hydroxypropylchitosan in einer 2. Reaktionsstufe mit einem Alkylhalogenid in alkalischem Medium bei einer Reaktionstemperatur von 40° bis 120 °C, vorzugsweise 60°–90 °C, zur Umsetzung bringt.

Das molare Verhältnis des Chitosans zu den Alkylierungsmitteln wählt man jeweils zwischen 1:3 bis 1:15.

Bei der gleichzeitigen Umsetzung des Chitosans mit beiden Alkylierungsmitteln wird das Reaktionsgemisch zunächst 3–60 Stunden lang bei 20° bis 120 °C, vorzugsweise 12–48 Stunden lang bei 20° bis 40 °C, im Autoklav gerührt. Unter diesen Reaktionsbedingungen findet vorzugsweise die N-Hydroxypropylierung statt. Anschliessend wird das Reaktionsgemisch 3–60 Stunden lang bei 40 bis 120 °C, vorzugsweise 6 bis 24 Stunden lang bei 60–90 °C, weitergerührt, wobei dann die O-Alkylierung abläuft.

Als Dispergiermittel genügen bei beiden Herstellungsverfahren, sofern die Reaktionstemperatur der 1. Reaktionsstufe bei 20° bis 40 °C liegt, die als Alkylierungsmittel enthaltenen überschüssigen Alkylhalogenide. Bei höheren Reaktionstemperaturen können zusätzlich organische Dispergiermittel, wie z.B. Ethanol, Isopropanol, Dioxan, Aceton und Toluol, Verwendung finden.

Als Alkylhalogenide werden die Chloride und Bromide von geradkettigen oder verzweigten Alkanen mit 1 bis 6 Kohlenstoffatomen eingesetzt.

Die Aufarbeitung der Reaktionsgemische erfolgt in der Weise, dass man nach Beseitigung des überschüssigen Propylenoxids das hydroxyalkylierte Zwischenprodukt durch Einengen aus dem organischen Lösungsmittel isoliert, bzw. nach der Alkylierung im alkalischen Medium das überschüssige Alkali durch Neutralisation entfernt, das Reaktionsgemisch einengt und das Chitosanderivat der Formel I durch Eingiessen in destilliertes Wasser ausfällt.

Die Salze der erfindungsgemässen alkyl-hydroxypropylsubstituierten Chitosane der allgemeinen Formel I können beispeilsweise durch Neutralisation der Aminogruppen der Chitosanderivate der Formel I mit Säuren erhalten werden. Gemäss der vorliegenden Erfindung sind jedoch nur solche Salze verwendbar, die in Wasser löslich sind. Geeignete Salze sind zum Beispiel solche mit den Säuren Salzsäure, Ameisensäure, Essigsäure, Milchsäure, Glykolsäure, Malonsäure, Zitronensäure und Adipinsäure.

Im Gegensatz zu synthetischen Polymeren mit endlichen Restmonomergehalten sind die vorstehend beschriebenen Chitosanderivate physiologisch unbedenklich, biologisch abbaubar und können analog zu den hochsubstituierten, organolöslichen, nichtionogenen Celluloseäthern in Klebstoffen und Lacken, in Pharmazeutika und Kosmetika sowie bei der Folien- und Filmherstellung zur Anwendung gelangen. Der zu ihrer Herstellung erforderliche Rohstoff Chitin steht zudem in den Weltmeeren als Bestandteil von Krustentieren und niederen Pflanzen in riesigen Mengen zur Verfügung, so dass eine Hinwendung zu diesem Rohstoff für den industriellen Einsatz sowohl aus Gründen des Umweltschutzes als auch aus ökonomischen Gesichtspunkten sinnvoll erscheint.

Mit den vorstehend beschriebenen Chitosanderivaten lassen sich insbesondere kosmetische Mittel zur Behandlung von Haaren oder der Haut herstellen, die sich durch überraschend vorteilhafte Eigenschaften auszeichnen und die dadurch gekennzeichnet sind, dass sie in einer geeigneten Kosmetikgrundlage eine makromolekulare, vom Chitosan abgeleitete polymere Verbindung der allgemeinen Formel I

$$HO\left[C_6H_{11-m-q}NO_4(R^1)_m\,(R^2)_n\,(R^3)_q\right]_pH \qquad (I),$$

wobei m jeden beliebigen Zahlenwert von 0 bis

0,6 bedeutet, n jeden beliebigen Zahlenwert von 0,5 bis 10 bedeutet, q ein beliebiger Zahlenwert von 0,5 bis 4 ist, p eine ganze Zahl von 50 bis 5000 bedeutet, $R^1$ ein Acetyl-Rest ist, $R^2$ einen zweiwertigen Rest

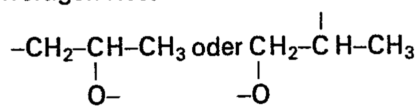
oder

darstellt und $R^3$ einen geradkettigen oder verzweigten Alkylrest mit 1 bis 6 Kohlenstoffatomen bedeutet, oder dessen Salz mit einer organischen oder anorganischen Säure enthalten.

Die erfindungsgemässe Chitosanderivate der Formel I enthaltenden Mittel eignen sich ganz allgemein zur Behandlung der Haut und/oder der Haare. Sie können beispielsweise vorliegen als Haar- und/oder Körperwaschmittel, Tönungsshampoos, Frisiercremes, Frisierlotionen, Mittel zur Festigung der Frisur, Waschlotionen, Fönlotionen, Haarkuren, Mittel gegen Kopfschuppen, Mittel zur permanenten Haarverformung, Mittel zur Färbung oder Entfärbung von Haaren, Mittel zum Auftragen vor oder nach der Haarfärbung und als kosmetische Mittel zur Pflege, zum Schutz oder zur Reinigung der Haut wie Gesichtswasser, Rasierwasser, Feuchthaltecremes, Coldcremes, Körperlotionen, Sonnenschutzmittel oder auch Make-up-Präparate wie Schminkcremes und Rouges.

Der Gehalt der hier beschriebenen kosmetischen Mittel an den neuen Chitosanderivaten der Formel I liegt zweckmässig bei 0,05 bis 10 Gew.-%, vorzugsweise bei 0,1 bis 6,0 Gew.-%.

Bevorzugter Gegenstand der vorliegenden Erfindung ist ein Mittel zur Festigung der Frisur in Form einer alkoholischen oder wässrig-alkoholischen Lösung des Chitosanderivates der Formel I, wobei das Chitosanderivat vorzugsweise nicht in Form des Salzes vorliegt. Die Mittel zur Festigung der Frisur enthalten das Chitosanderivat vorzugsweise in einer Menge von 0,5 bis 5 Gew.-%.

Die kosmetischen Mittel gemäss der vorliegenden Erfindung können zusätzlich zu dem neuen Chitosanderivat der Formel I zur Herstellung einer Kosmetikgrundlage alle diejenigen Bestandteile enthalten, die in Haar- und Hautbehandlungsmitteln üblicherweise eingesetzt werden, insbesondere anionische, kationische, amphotere, zwitterionische oder nichtionische oberflächenaktive Tenside, Schaumsynergisten Stabilisatoren, Sequestriermittel, Pigmente, Verdicker, Emulgatoren, Pufferstoffe, Konservierungsmittel, Farbstoffe, Parfümöle, bekannte kosmetische Polymere wie anionische, nichtionische, kationische oder amphotere Polymere, Naturstoffe, kosmetische Öle, Fettalkohole, Wachse, Schaumstabilisatoren, Wirkstoffe gegen Kopfschuppen, Reduktionsmittel und Treibmittel.

Die erfindungsgemässen kosmetischen Mittel weisen in bevorzugter Weise einen pH-Wert von 2 bis 11 auf und können in Form wässriger, alkoholischer oder wässrig-alkoholischer Zubereitungen, z.B. mit einem Alkohol mit 1 bis 4 Kohlenstoffatomen, als Lösungen, als Cremes, als Gele, als Dispersionen oder als Emulsionen vorliegen. Ebenfalls ist es möglich, diese Mittel in Form der alkoholischen oder wässrig-alkoholischen Lösung mit Hilfe eines Zerstäubers beziehungsweise anderer geeigneter Sprühvorrichtungen oder im Gemisch mit üblichen unter Druck verflüssigten Treibmitteln als Aerosolhaarspray aus einem Druckbehälter zu versprühen.

Wenn es sich bei den erfindungsgemässen kosmetischen Mitteln um Mittel zur Festigung der Frisur, wie flüssige Haarfestiger oder Haarsprays handelt, dann liegen sie üblicherweise als alkoholische oder wässrig-alkoholische Lösungen vor, die durch einen Gehalt an Chitosanderivaten der vorstehend genannten Formel I gekennzeichnet sind. Hierbei können die Chitosanderivate allein als filmbildendes beziehungsweise festigendes Harz eingesetzt werden. Es können jedoch auch zusätzlich bekannte filmbildende natürliche oder synthetische Polymere in dem erfindungsgemässen Haarfestigungsmittel enthalten sein. Als natürliche Polymere kommen beispielsweise Schellack, Alginate, Gelatine, Pektine und Cellulosederivate in Betracht. Von den synthetischen Polymeren finden z.B. Polyvinylpyrrolidon, Polyvinylacetat, Polyacrylverbindungen, wie Acrylsäure- oder Methacrylsäurepolymerisate, basische Polymerisate von Estern aus Acrylsäure oder Methacrylsäure mit Aminoalkoholen beziehungsweise die Salze oder Quaternisierungsprodukte dieser basischen Polymerisate, Polyacrylnitril sowie Co- oder Terpolymerisate aus derartigen Verbindungen, beispielsweise Polyvinylpyrrolidon-Vinylacetat, Verwendung. Die Mittel weisen dann insbesondere einen pH-Wert zwischen 6 und 8 auf. Solche Mittel zur Festigung der Frisur enthalten üblicherweise filmbildenden Polymere in einer Gesamtmenge von etwa 0,05 bis 3,0 Gew.-%. Enthalten die Mittel neben den quaternären Chitosanderivaten der Formel I noch andere filmbildende Polymere, so reduziert sich der Gehalt an quaternären Chitosanderivaten entsprechend.

Als Lösungsmittel kommen, neben Alkoholen wie z.B. Ethanol und Isopropanol, auch Ketone wie Aceton, Halogenkohlenwasserstoffe wie beispielsweise Dichlormethan sowie ferner Kohlenwasserstoffe und niedere Silikonöle in Betracht.

Da die erfindungsgemässen neuen Chitosanderivate, sofern sie nicht als Salz vorliegen, gut in organischen Lösungsmitteln löslich sind, können sie auch in wasserfreien Mitteln zur Festigung von Haaren eingesetzt werden.

Wenn die Mittel zur Festigung der Frisur in Form von Aerosolpräparaten vorliegen, welche aus einem Druckbehälter versprüht werden, so enthalten sie in der Kosmetikgrundlage etwa 10 bis 60 Gew.-% eines Treibmittels. Als Treibmittel können Chlorfluoralkane, wie z.B. $CCl_3F$, $CCl_2F_2$, $C_2Cl_3F_3$, $CCl_2F-CCl_2F$, $CHCl_2F$, $CHClF_2$ und $CClF_2-CClF_2$, leichtflüchtige Kohlenwasserstoffe, wie z.B. n-Butan und n-Propan, oder auch Dimethylether, $CO_2$, $N_2O$, $N_2$, $CH_2Cl_2$ und $CCl_3-CH_3$ Verwendung finden.

Die hier beschriebenen Mittel zur Festigung der Frisur können weiterhin die für solche Mittel üblichen Zusätze, beispielsweise Modifiziermittel, wie z.B. Siliconöl, oder Weichmacher, wie z.B. Isopropylmyristat, Phthalsäurediethylester und Diethylstearat, sowie ferner kämmbarkeitsverbessernde Substanzen, enthalten.

Die erfindungsgemässen Mittel zur Festigung der Frisur können gegebenenfalls durch einen Gehalt an kosmetischen Farbstoffen das Haar gleichzeitig färben oder tönen. Derartige Präparate sind u.a. als Farbfestiger oder Tönungsfestiger im Handel bekannt. Sie enthalten zusätzlich übliche für Haarfestiger bekannte Farbstoffe wie beispielsweise aromatische Nitrofarbstoffe (z.B. 1,4-Diamino-2-nitro-benzol), Azofarbstoffe (z.B. C.I. Acid Brown 4), Anthrachinonfarbstoffe (z.B. C.I. Disperse Violet 4) und Triphenylmethanfarbstoffe (z.B. C.I. Basic Violet 1), wobei die Farbstoffe dieser Klassen je nach der Art ihrer Substituenten sauren, nichtionogenen oder basischen Charakter haben können. Ihre Gesamtkonzentration in diesen Präparaten beträgt üblicherweise etwa 0,01 bis 2,0 Gew.-%.

Die erfindungsgemässen kosmetischen Mittel können weiterhin Mittel zur Festigung von Haaren ohne Treibmittel darstellen. Hierzu werden sie in flüssiger Form vor dem Einlegen oder Fönen auf das Haar aufgetragen.

Die nachfolgenden Beispiele sollen den Gegenstand der Erfindung näher erläutern.

Herstellungsbeispiele

Beispiel 1

1. Stufe:

*N-Hydroxypropylierung von Chitosan*

50 g (0,31 Mol) Chitosan ($\eta$ = 140 ml/g; freies Amin 86%; Teilchengrösse < 25 µm) werden zusammen mit 100 ml destilliertem $H_2O$, 100 ml Ethanol und 209,08 g ≙ 250 ml (3,6 Mol) Propylenoxid in einen Autoklaven eingebracht und 12 Stunden lang bei 100 °C gerührt.

Nach beendeter Reaktion verdünnt man die hochviskose, braune Masse mit einem Gemisch aus Ethanol/$H_2O$ 1:1, bringt zur Entfernung eventuell vorhandener Reste an Propylenoxid die Lösung unter dem Abzug kurzzeitig zum Sieden und führt vor dem Einengen am Rotationsverdampfer, falls zur Klärung notwendig, eine Druckfiltration durch.

Man trocknet zunächst an der Luft, später bei 50 °C im Vakuumtrockenschrank und erhält ein Hydroxypropylchitosan in einer Ausbeute von 92% der Theorie unter der Annahme einer erfolgten Disubstitution.

Kenndaten des Reaktionsproduktes:
Grenzviskositätszahl: $[\eta]^{25\,°C}$ = 49 ml/g (gemessen in wässriger 0,2 m Essigsäure/0,1 m Natriumacetat-Lösung)
Substitutionsgrad Hydroxypropyl (n): 1,70
Substitutionsgrad Acetyl (m): 0,22

2. Stufe:

*O-Ethylalkylierung von N-Hydroxypropyl-Chitosan*

83,1 g (0,3 Mol) des N-Hydroxypropyl-Chitosans aus der obigen Umsetzung werden zusammen mit 40 ml 43%-iger NaOH, 400 ml Aceton sowie 327,0 g ≙ 225 ml (3,0 Mol) Ethylbromid in den Autoklav eingebracht und 12 Stunden lang bei 60 °C gerührt.

Nach Beendigung der Reaktion stellt man die viskose, schwach gelb gefärbte Lösung mittels konzentrierter HCl auf pH 7 ein, engt mit dem Rotationsverdampfer bis zur Trockene ein und trennt durch Aufnahme in absolutem Ethanol den Hauptteil der anorganischen Nebenprodukte ab.

Das ethanolische Filtrat trägt man unter intensivem Rühren in destilliertes Wasser ein, sammelt den Niederschlag, wäscht halogenidfrei und trocknet ihn bei 50 °C im Vakuumtrockenschrank.

Zur weiteren Reinigung kann man das isolierte Produkt in 2 l Methylenchlorid lösen, über $CaCl_2$ trocknen, die Lösung druckfiltrieren und nach dem Einengen an der Luft beziehungsweise bei 50 °C im Vakuumtrockenschrank trocknen.

Der resultierende transparente, hochglänzende Polymerfilm wird in einer Schlagkreuzmühle zerkleinert. Das so erhaltene Chitosanderivat verfügt über folgende Kenndaten:
Grenzviskositätszahl: $[\eta]^{25\,°C}_{THF}$ = 33 ml/g
Substitutionsgrad Ethyl (q): 2,30
Substitutionsgrad Hydroxypropyl (n): 1,70
Substitutionsgrad Acetyl (m): 0,22

Beispiel 2

*O-Butylalkylierung von N-Hydroxypropyl-Chitosan*

50 g (0,181 Mol) des aus der 1. Stufe von Beispiel 1 erhaltenen N-Hydroxypropyl-Chitosans werden zusammen mit 80,8 ml 43%iger NaOH, 235 ml Aceton sowie 248 g ≙ 195 ml (1,81 Mol) 1-Brombutan in einen Autoklav eingebracht und 24 Stunden lang bei 90 °C gerührt.

Die Aufarbeitung und Isolierung erfolgt in der für die 2. Stufe in Beispiel 1 beschriebenen Weise.
Kenndaten des Chitosanderivates:
Grenzviskositätszahl: $[\eta]^{25\,°C}_{THF}$ = 22 ml/g
Substitutionsgrad Butyl (q): 3,54
Substitutionsgrad Hydroxypropyl (n): 1,70
Substitutionsgrad Acetyl (m): 0,22

Beispiel 3

*O-Hexylalkylierung von N-Hydroxypropyl-Chitosan*

50 g (0,181 Mol) des nach Beispiel 1 hergestellten N-Hydroxypropyl-Chitosans werden zusammen mit 80,8 ml 43%iger NaOH, 235 ml Aceton sowie 109,16 ≙ 95 ml (0,905 Mol) 1-Chlorhexan in einen Autoklaven eingebracht und 18 Stunden lang bei 90 °C gerührt.

Nach Aufarbeitung und Isolierung, wie in Beispiel 1 beschrieben, erhält man ein transparentes, weiches, flexibles Mischätherderivat des Chitosans.
Kenndaten des Chitosanderivates:
Grenzviskositätszahl: $[\eta]^{25\,°C}_{THF}$ = 14 ml/g
Substitutionsgrad Hexyl (q): 1,46
Substitutionsgrad Hydroxypropyl (n): 1,70
Substitutionsgrad Acetyl (m): 0,22

Beispiel 4

*Gleichzeitige Umsetzung des Chitosans mit Propylenoxid und einem Alkylhalogenid (Ethylchlorid)*

20 g (0,12 Mol) Chitosan ($\eta$ = 140 ml/g; freies Amin 86%; Teilchengrösse < 25 µm) werden mit 325 ml 43%iger NaOH versetzt, durch jeweils 3-maliges Gefrieren während 30 Minuten und anschliessendes Auftauen während 60 Minuten in Alkalichitosan umgewandelt.

Man saugt das Alkalichitosan bis auf ein Feuchtgewicht von 160–165 g ab, bringt es zusammen mit 174,2 g ≙ 210 ml (3,0 Mol) Propylenoxid sowie 193,6 g ≙ 211 ml (3,0 Mol) Ethylchlorid in einen Autoklav ein und lässt das Gemisch zunächst 12 Stunden lang bei 35 °C und dann weitere 12 Stunden lang bei 90 °C unter Rühren reagieren. Nach beendeter Reaktion verdünnt man die viskose Lösung mit einem Gemisch aus Aceton/$H_2O$, entfernt die überschüssigen, nicht umgesetzten Alkylierungsmittel und stellt mittels konzentrierter HCl auf pH 7 ein. Durch Einengen bis zur Trockene und anschliessendes Aufnehmen in Aceton trennt man die Hauptmenge des anorganischen Salzes ab. Das acetonische Filtrat trägt man unter intensivem Rühren in destilliertes $H_2O$ ein, sammelt den Niederschlag, wäscht halogenidfrei und trocknet ihn bei 50 °C im Vakuumtrockenschrank.

Das erhaltene Chitosanderivat verfügt über folgende Kennzahlen:

Grenzviskositätszahl: $[\eta]_{THF}^{25\,°C}$ = 44 ml/g

Substituitionsgrad Hydroxypropyl (n): 1,76

Substitutionsgrad Ethyl (q): 2,40

Der Substitutionsgrad für die Hydroxypropyl- und Alkylreste wurde jeweils mit Hilfe der $^1$H-NMR-Spektren bestimmt.

Beispiele für kosmetische Mittel

Die Beispiele 5–17 stellen Aerosolhaarsprays dar. In allen Fällen wurden Haarsprays mit hervorragenden anwendungstechnischen Eigenschaften erhalten, die im Vergleich zu handelsüblichen Haarsprays insbesondere einen natürlicheren Griff der Haare, eine kürzere Trockenzeit sowie ein natürlicheres Aussehen der Frisur bewirken.

Die Beispiele 18–28 zeigen die Möglichkeit zur gemeinsamen Verwendung der erfindungsgemässen Chitosanderivate mit bekannten handelsüblichen Haarsprayharzen für Aerosolhaarsprays. In allen Fällen wurden Haarsprays mit guten anwendungstechnischen Eigenschaften erhalten, die im Vergleich zu handelsüblichen Haarsprays über eine leichtere Auskämmbarkeit und eine durch niedrigere statische Aufladung verbesserte Frisierbarkeit verfügen.

Die Beispiele 29–34 zeigen den Einsatz der erfindungsgemässen kosmetischen Mittel als Non-Aerosol-Haarsprays, das heisst Mittel zur Festigung der Frisur ohne Treibmittel, welche dazu bestimmt sind, mittels einer Sprühpumpe auf das Haar aufgetragen zu werden. Bei allen Rezepturen wird nach der Anwendung ein guter Halt der Frisur bei natürlichem Griff und Aussehen erhalten.

Die Beispiele 35 und 36 zeigen die Anwendung der erfindungsgemässen Mittel als Haareinlegemittel. 15 ml der Haareinlegemittel werden vor dem Wickeln auf das Haar aufgetragen und verteilt. Nach Einlegen und Trocknen wird eine gute Festigung der erhaltenen Frisur erreicht.

Beispiele 5–17: Aerosolhaarsprays (Alle Angaben in Gew.-%)

| Beispiel Nr. | 5 | 6 | 7 | 8 | 9 | 10 | 11 | 12 | 13 | 14 | 15 | 16 | 17 |
|---|---|---|---|---|---|---|---|---|---|---|---|---|---|
| Chitosanderivat nach Beispiel 1 | 2,65 | 2,65 | 2,65 | 2,65 | 2,65 | 2,65 | 2,65 | 2,65 | 2,65 | 2,65 | 2,65 | 2,65 | 2,65 |
| Parfümöl | 0,18 | 0,18 | 0,18 | 0,18 | 0,18 | 0,18 | 0,18 | 0,18 | 0,18 | 0,18 | 0,18 | 0,18 | 0,18 |
| Siliconöl | 0,04 | 0,04 | 0,04 | 0,04 | 0,04 | 0,04 | 0,04 | 0,04 | 0,04 | 0,04 | 0,04 | 0,04 | 0,04 |
| Isopropanol | 57,13 | 22,13 | – | – | 57,13 | 22,13 | – | – | 12,14 | – | 12,14 | – | 12,14 |
| Methylenchlorid | – | 35,00 | – | 35,00 | – | 35,00 | – | 35,00 | 35,00 | 35,00 | 35,00 | 35,00 | 35,00 |
| Ethanol | – | – | 57,13 | 22,13 | – | – | 57,13 | 22,13 | – | 12,14 | – | 12,14 | – |
| Aceton | – | – | – | – | – | – | – | – | 9,99 | 9,99 | 9,99 | 9,99 | 9,99 |
| Summe | 60,00 | 60,00 | 60,00 | 60,00 | 60,00 | 60,00 | 60,00 | 60,00 | 60,00 | 60,00 | 60,00 | 60,00 | 60,00 |
| Treibmittel 50:50 Gemisch aus $CCl_3F$ und $CCl_2F_2$ | 40,00 | 40,00 | 40,00 | 40,00 | – | – | – | – | 40,00 | 40,00 | – | – | – |
| Gemisch aus Propan/Butan im Verhältnis 80:20 | – | – | – | – | 40,00 | 40,00 | 40,00 | 40,00 | – | – | 40,00 | 40,00 | 20,00 |
| $CCl_3F$ | – | – | – | – | – | – | – | – | – | – | – | – | 20,00 |
| | 100,00 | 100,00 | 100,00 | 100,00 | 100,00 | 100,00 | 100,00 | 100,00 | 100,00 | 100,00 | 100,00 | 100,00 | 100,00 |

Beispiele 18–28: Aerosolhaarsprays mit Harzmischungen (Alle Angaben in Gew.-%)

| Beispiel Nr. | 18 | 19 | 20 | 21 | 22 | 23 | 24 | 25 | 26 | 27 | 28 |
|---|---|---|---|---|---|---|---|---|---|---|---|
| Chitosanderivat nach Beispiel 2 | 2,50 | 2,50 | 2,50 | 2,50 | 2,50 | 1,50 | 1,50 | 1,50 | 1,50 | 1,50 | 1,50 |
| Copolymer aus Vinylpyrrolidon/Vinylacetat (30:70), 50%ige isopropanolische Lösung | 0,50 | – | – | 0,50 | 0,50 | 1,50 | – | – | – | 1,50 | 1,50 |
| Copolymer aus Vinylpyrrolidon/Vinylacetat (30:70), 50%ige ethanolische Lösung | – | – | – | – | – | – | 1,50 | – | – | – | – |
| Copolymer aus Vinylacetat/Crotonsäure (90:10) fest | – | 0,50 | – | – | – | – | – | 1,50 | – | – | – |
| Copolymer aus Octylacrylamid/Acrylsäure/Butylamino-ethylmethacrylat, fest (AMPHOMER® of National Starch Chem. Corp. USA) | – | – | 0,50 | – | – | – | – | – | 1,50 | – | – |
| Parfümöl | 0,18 | 0,18 | 0,18 | 0,18 | 0,18 | 0,18 | 0,18 | 0,18 | 0,18 | 0,18 | 0,18 |
| Siliconöl | 0,04 | 0,04 | 0,04 | 0,04 | 0,04 | 0,04 | 0,04 | 0,04 | 0,04 | 0,04 | 0,04 |
| Isopropanol oder Ethanol | 56,78 | 56,78 | 56,78 | 21,78 | 11,78 | 56,78 | 56,78 | 56,78 | 56,78 | 21,78 | 11,78 |
| Methylenchlorid | – | – | – | 35,00 | 35,00 | – | – | – | – | 35,00 | 35,00 |
| Aceton | – | – | – | – | 10,00 | – | – | – | – | – | 10,00 |
| 50:50-Gemisch aus $CCl_3F$ und $CCl_2F_2$ | – | – | – | – | – | 40,00 | 40,00 | 40,00 | 40,00 | 40,00 | 40,00 |
| Gemisch aus Propan/Butan im Verhältnis 80:20 | 40,00 | 40,00 | 40,00 | 40,00 | 40,00 | – | – | – | – | – | – |
| | 100,00 | 100,00 | 100,00 | 100,00 | 100,00 | 100,00 | 100,00 | 100,00 | 100,00 | 100,00 | 100,00 |

Beispiele 29–34: Non-Aerosol-Haarsprays (Haarsprays ohne Treibgas) (Alle Angaben in Gew.-%)

| Beispiel Nr. | 29 | 30 | 31 | 32 | 33 | 34 |
|---|---|---|---|---|---|---|
| Chitosanderivat nach Beispiel 1 | 3,00 | 3,00 | 3,00 | – | – | – |
| Chitosanderivat nach Beispiel 4 | – | – | – | 2,50 | 0,50 | 2,50 |
| Copolymer aus Vinylpyrrolidon/Vinylacetat (30:70), 50%ige alkoholische Lösung | – | – | – | 0,50 | 2,50 | 0,50 |
| Silikonöl | 0,05 | 0,05 | 0,05 | 0,05 | 0,05 | 0,05 |
| Isopropanol | 60,00 | – | 40,00 | 60,00 | – | 40,00 |
| Ethanol | – | 60,00 | – | – | 60,00 | – |
| Aceton | – | – | 20,00 | – | – | 20,00 |
| Parfümöl | 0,20 | 0,20 | 0,20 | 0,20 | 0,20 | 0,20 |
| Wasser | 36,75 | 36,75 | 36,75 | 36,75 | 36,75 | 36,75 |
| | 100,00 | 100,00 | 100,00 | 100,00 | 100,00 | 100,00 |

Beispiele 35 und 36: Haareinlegemittel

| Beispiel Nr. | 35 | 36 |
|---|---|---|
| Chitosanderivat nach Beispiel 1 | 2,88 | 1,44 |
| Copolymer aus Vinylacetat/Crotonsäure/ Ethylenoxid, 60%ige isopropanolische Lösung (CAS Nr. 25 609-89-6) | – | 4,80 |
| Isopropanol | 2,60 | 2,60 |
| Ethanol | 45,00 | 45,00 |
| Parfümöl | 0,40 | 0,40 |
| Phthalsäurediethylester | 0,20 | 0,20 |
| Wasser | 48,92 | 45,56 |
| | 100,00 | 100,00 |

(Alle Angaben in Gew.-%)

## Patentansprüche

1. Kosmetisches Mittel zur Behandlung von Haaren oder der Haut, dadurch gekennzeichnet, dass es in einer geeigneten Kosmetikgrundlage eine makromolekulare, vom Chitosan abgeleitete polymere Verbindung der allgemeinen Formel I

$$HO\left[C_6H_{11-m-q}NO_4(R^1)_m(R^2)_n(R^3)_q\right]_pH \qquad (I),$$

wobei m jeden beliebigen Zahlenwert von 0 bis 0,6 bedeutet, n jeden beliebigen Zahlenwert von 0,5 bis 10 bedeutet, q ein beliebiger Zahlenwert von 0,5 bis 4 ist, p eine ganze Zahl von 50 bis 5000 bedeutet, $R^1$ ein Acetyl-Rest ist, $R^2$ einen zweiwertigen Rest

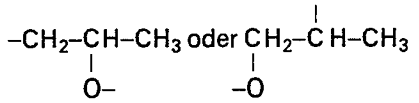

darstellt und $R^3$ einen geradkettigen oder verzweigten Alkylrest mit 1 bis 6 Kohlenstoffatomen bedeutet, oder dessen Salz mit einer organischen oder anorganischen Säure enthält.

2. Mittel nach Anspruch 1, dadurch gekennzeichnet, dass n in der Formel I einen Zahlenwert von 1 bis 3 darstellt.

3. Mittel nach Anspruch 1 und 2, dadurch gekennzeichnet, dass q in der Formel I einen Zahlenwert von 1 bis 3,6 bedeutet.

4. Mittel nach Anspruch 1 bis 3, dadurch gekennzeichnet, dass es die polymere Verbindung der Formel I in einer Menge von 0,05 bis 10 Gew.-% enthält.

5. Mittel nach Anspruch 1 bis 4, dadurch gekennzeichnet, dass die Kosmetikgrundlage eine wässrige, alkoholische oder wässrig-alkoholische Lösung, eine Creme ein Gel oder eine Emulsion ist.

6. Mittel nach Anspruch 1 bis 5, dadurch gekennzeichnet, dass es als Kosmetikgrundlage eine alkoholische oder wässrig-alkoholische Lösung eines niedermolekularen Alkohols, wie Ethanol oder Isopropanol, umfasst, einen pH-Wert zwischen 6 und 8 aufweist und ein Mittel zur Festigung der Frisur darstellt.

7. Mittel näch Anspruch 1 bis 6, dadurch gekennzeichnet, dass die Kosmetikgrundlage eine alkoholische oder wässrig-alkoholische Lösung ist, die mit einem unter Druck verflüssigten Treibgas vermischt ist, in einem Druckbehälter abgefüllt ist und in Form eines Aerosolhaarsprays vorliegt.

8. Mittel nach Anspruch 1 bis 7, dadurch gekennzeichnet, dass es zusätzlich ein bekanntes

filmbildendes synthetisches oder natürliches kosmetisches Polymer enthält.

9. Mittel nach Anspruch 1 bis 7, dadurch gekennzeichnet, dass die Kosmetikgrundlage eine wässrige, alkoholische oder wässrig-alkoholische Lösung, ein Gel oder eine Emulsion ist, welche zusätzlich einen Farbstoff enthält und in Form eines Farbfestigers oder Tönungsfestigers vorliegt.

10. Makromolekulare, vom Chitosan abgeleitete polymere Verbindung der allgemeinen Formel I

$$HO\left[C_6H_{11\text{-}m\text{-}q}NO_4(R^1)_m(TR^2)_n(R^3)_q\right]_pH \quad (I),$$

wobei m jeden beliebigen Zahlenwert von 0 bis 0,6 bedeutet, n jeden beliebigen Zahlenwert von 0,5 bis 10 bedeutet, q ein beliebiger Zahlenwert von 0,5 bis 4,0 ist, p eine ganze Zahl von 50 bis 5000 bedeutet, $R^1$ ein Acetyl-Rest ist, $R^2$ einen zweiwertigen Rest

$$-CH_2-\underset{\displaystyle O-}{\underset{|}{C}}H-CH_3 \text{ oder } \underset{\displaystyle -O}{\underset{|}{C}}H_2-\overset{|}{C}H-CH_3$$

darstellt und $R^3$ einen geradkettigen oder verzweigten Alkylrest mit 1 bis 6 Kohlenstoffatomen bedeutet, oder dessen Salz mit einer organischen oder anorganischen Säure.

11. Verbindung gemäss Anspruch 10, dadurch gekennzeichnet, dass in der Formel I n einen Zahlenwert 1 bis 3 bedeutet, q einen Zahlenwert von 1 bis 3,6 darstellt und die Grenzviskositätszahl einen Wert von 14 bis 44 ml/g aufweist.

12. Verbindung nach Patentanspruch 10, aufgebaut aus

4 bis 50 Mol-% Einheiten der allgemeinen Formel

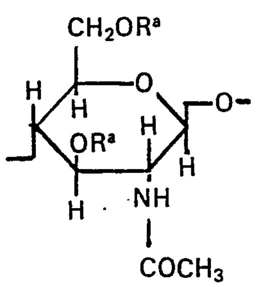

(A),

40 bis 96 Mol-% Einheiten der allgemeinen Formel

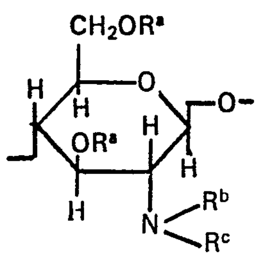

(B) und

0 bis 10 Mol-% Einheiten der allgemeinen Formel

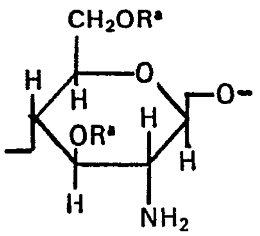

(C)

in statistischer Verteilung, wobei $R^a$ die Bedeutung H oder $C_1$–$C_6$-Alkyl hat, $R^b$ einen Rest H oder

$$\left[-CH_2-\underset{\displaystyle CH_3}{\underset{|}{C}}H-O-\ -R-^a,\right]_x$$

mit x = eine ganze Zahl von 1 bis 5, darstellt und $R^c$ die Bedeutung

$$\left[-CH_2-\underset{\displaystyle CH_3}{\underset{|}{C}}H-O-\right]_x-R^a$$

hat, unter der Voraussetzung, dass mindestens einer der Substituenten $R^a$ in den Formeln A oder B $C_1$–$C_6$-Alkyl bedeutet.

13. Verfahren zur Herstellung der Verbindungen nach Anspruch 10 bis 12, dadurch gekennzeichnet, dass man ein Chitosan, bestehend aus zu 50 bis 96% entacetyliertem Chitin, in einer 1. Reaktionsstufe in Gegenwart eines organischen Dispergiermittels bie einer Temperatur von 20 °C bis 120 °C mit Propylenoxid umsetzt und das erhaltene Hydroxypropyl-Chitosan in einer 2. Reaktionsstufe mit einem Alkylhalogenid in alkalischem Medium bei einer Reaktionstemperatur von 40° bis 120 °C zur Umsetzung bringt, wobei das molare Verhältnis zwischen Chitosan und Alkylierungsmittel jeweils zwischen 1:3 und 1:15 beträgt.

## Revendications

1. Produit cosmétique de traitement des cheveux ou de la peau, caractérisé en ce qu'il renferme, dans un substrat cosmetique approprié, un composé macromoléculaire polymère dérivé du chitosane, repondant à la formule générale (I):

$$HO\left[C_6H_{11\text{-}m\text{-}q}NO_4(R^1)_m(R^2)_n(R^3)_q\right]_pH \quad (I)$$

m représentant n'importe quelle valeur numérique de 0 à 0,6, n n'importe quelle valeur numérique de 0,5 à 10, q une valeur numérique quelconque de 0,5 à 4, p un nombre entier de 50 à 5000, $R^1$ un reste acétyle, $R^2$ un reste bivalent:

$$-CH_2-\underset{\displaystyle O-}{\underset{|}{C}}H-CH_3 \text{ ou } \underset{\displaystyle -O}{\underset{|}{C}}H_2-CH-CH_3$$

et $R^3$ un reste alkyle linéaire ou ramifié de 1 à 6 atomes de carbone, ou son sel avec un acide organique ou inorganique.

2. Produit selon la revendication 1, caractérisé

en ce que, dans la formule (I), n représente une valeur numérique de 1 à 3.

3. Produit selon les revendications 1 et 2, caractérisé en ce que, dans la formule (I), q représente un nombre entier de 1 à 3,6.

4. Produit selon l'une quelconque des revendications 1 à 3, caractérisé en ce qu'il renferme le composé polymère répondant à la formule (I) dans une proportion de 0,05 à 10% en poids.

5. Produit selon l'une quelconque des revendications 1 à 4, caractérisé en ce que le substrat cosmétique est une solution aqueuse, alcoolique ou hydro-alcoolique, une crème, un gel ou une émulsion.

6. Produit selon l'une quelconque des revendications 1 à 5, caractérisé en ce qu'il renferme, comme substrat cosmétique, une solution alcoolique ou hydro-alcoolique d'un alcool inférieur, comme l'éthanol ou l'isopropanol, en ce qu'il présente une valeur de pH comprise entre 6 et 8 et en ce qu'il constitue un produit de fixation de la coiffure.

7. Produit selon l'une quelconque des revendications 1 à 6, caractérisé en ce que le substrat cosmetique est une solution alcoolique ou hydroalcoolique melangée avec un gaz propulseur liquéfié sous pression, contenue dans un récipient pressurisé et se présentant sous la forme d'un spray capillaire à aérosol.

8. Produit selon l'une quelconque des revendications 1 à 7, caractérisé en ce qu'il renferme, en plus, un polymère cosmétique synthétique ou naturel filmogène connu.

9. Produit selon l'une quelconque des revendications 1 à 7, caractérisé en ce que le substrat cosmétique est une solution aqueuse, alcoolique ou hydro-alcoolique, un gel ou une émulsion, qui renferme en plus un colorant et qui se présente sous la forme d'un fixateur de couleur ou d'un fixateur de nuance.

10. Composé macromoléculaire polymère dérivé du chitosane répondant à la formule générale (I):

$$HO\left[C_6H_{11-m-q}NO_4(R^1)_m(R^2)_n(R^3)_q\right]_pH \quad (I)$$

dans laquelle m représente n'importe qu'elle valeur numérique de 0 à 0,6, n n'importe quelle valeur numérique de 0,5 à 10, q une valeur numérique quelconque de 0,5 à 4,0, p un nombre entier de 50 à 5000, $R^1$ un reste acétyle, $R^2$ un reste bivalent:

$$-CH_2-\underset{O-}{\underset{|}{C}}H-CH_3 \text{ ou } \underset{-O}{\underset{|}{C}}H_2-\overset{|}{C}H-CH_3$$

et $R^3$ un reste alkyle linéaire ou ramifié de 1 à 6 atomes de carbone, ou son sel avec un acide organique ou inorganique.

11. Composé selon la revendication 10, caractérisé en ce que, dans la formule (I), n représente une valeur numérique de 1 à 3, q une valeur numérique de 1 à 3,6, et en ce que l'indice de viscosité limité présente une valeur de 14 à 44 ml/g.

12. Composé selon la revendication 10, constitué par un pourcentage molaire de 4 à 50% de groupes répondant à la formule générale:

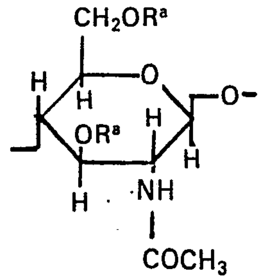

(A)

un pourcentage molaire de 40 à 96% de groupes répondant à la formule générale:

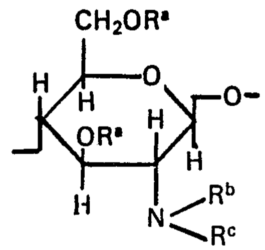

(B)

et un pourcentage molaire de 0 à 10% de groupes répondant à la formule générale:

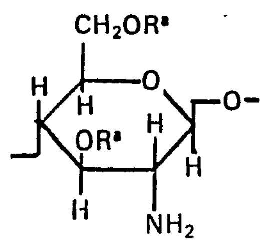

(C)

selon une distribution statistique, $R^a$ représentant H ou un alkyle en $C_1$ à $C_6$, $R^b$ H ou un reste

$$\left[-CH_2-\underset{CH_3}{\underset{|}{C}}H-O-\right]_x-R^a$$

avec x = nombre entier de 1 à 5, et $R^c$ représentant:

$$\left[-CH_2-\underset{CH_3}{\underset{|}{C}}H-O-\right]_x-R^a$$

sous la condition que l'un au moins des substituants $R^a$ dans les formules A ou B représente un alkyle en $C_1$ à $C_6$.

13. Procédé de préparation des composés selon l'une quelconque des revendications 10 à 12, caractérisé en ce qu'on fait réagir un chitosane comprenant de la chitine désacétylée à 50 à 96%, au cours d'un premier stade de réaction, en présence d'un agent dispersant organique, à une température de 20 °C à 120 °C, sur de l'oxyde de propylène, et l'on fait réagir l'hydroxypropyl-chitosane obtenu, au cours d'un second stade de réaction, sur un halogénure d'alkyle, en milieu alcalin, à une température de réaction de 40 à 120 °C, la proportion molaire de chitosane à l'agent d'alkylation étant entre 1:3 et 1:15.

## Claims

1. Cosmetic medium for treating hair or skin, characterised in that it contains, in a suitable cosmetic base, a macromolecular polymeric compound derived from chitosan, of the general formule I

$$HO\left[C_6H_{11\text{-}m\text{-}q}NO_4(R^1)_m(R^2)_n(R^3)_q\right]_pH \quad (I),$$

wherein m has any numerical value from 0 to 0.6, n has any numerical value from 0.5 to 10, q has any numerical value from 0.5 to 4, p is an integer from 50 to 5000, $R^1$ is an acetyl residue, $R^2$ is a bivalent residue

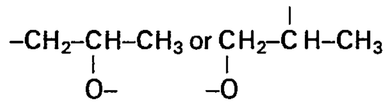

and $R^3$ contains a straight-chain or branched alkyl residue with 1 to 6 carbon atoms, or a salt thereof with an organic or inorganic acid.

2. Medium according to Claim 1, characterised in that n in formula I has a numerical value from 1 to 3.

3. Medium according to Claim 1 and 2, characterised in that q in formula 1 has a numerical value from 1 to 3.6.

4. Medium according to Calim 1 to 3, characterised in that it contains the polymeric compound of formula I in a quantity of 0.05 to 10 weight %.

5. Medium according to Claim 1 to 4, characterised in that the cosmetic base is an aqueous, alcoholic or aqueous-alcoholic solution, a cream, a gel or an emulsion.

6. Medium according to Claim 1 to 5, characterised in that it contains as cosmetic base, an alcoholic or aqueous-alcoholic solution of a low molecular weight alcohol, such as ethanol or isopropanol, has a pH value between 6 and 8 and is a medium for strengthening hair.

7. Medium according to Claim 1 to 6, characterised in that the cosmetic base is an alcoholic or aqueous-alcoholic solution, which is mixed with a propellant gas liquefied under pressure, charged into a pressure container and is in the form of an aerosol hair spray.

8. Medium according to Claim 1 to 7, characterised in that it also contains a known film-forming synthetic or natural cosmetic polymer.

9. Medium according to Claim 1 to 7, characterised in that the cosmetic base is an aqueous, alcoholic or aqueous-alcoholic solution, a gel or an emulsion, which also contains a dye and is in the form of a colour or tone strengthener.

10. Macromolecular polymeric compound derived from chitosan of the general formula I

$$HO\left[C_6H_{11\text{-}m\text{-}q}NO_4(R^1)_m(R^2)_n(R^3)_q\right]_pH \quad (I),$$

wherein m has any numerical value from 0 to 0.6, n has any numerical value from 0.5 to 10, q has any numerical value from 0.5 to 4.0, p is an integer from 50 to 5000, $R^1$ is an acetyl residue, $R^2$ is a bivalent residue

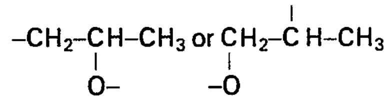

and $R^3$ is a straight chain or branched alkyl residue with 1 to 6 carbon atoms, or a salt thereof with an organic or inorganic acid.

11. Compound according to Claim 10, characterised in that in formula I n has a numerical value between 1 and 3, q has a numerical value from 1 to 3.6 and the limiting viscosity has a numerical value from 14 to 44 ml/g.

12. Compound according to Claim 10, formed from 4 to 50 mol-% of units of the general formula

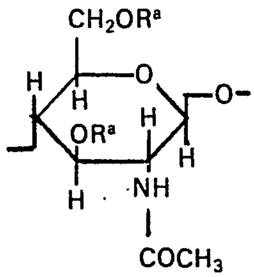

(A),

40 to 96 mol-% of units of the general formula

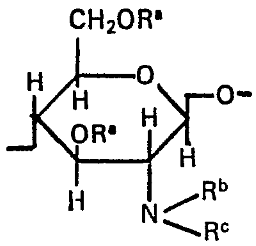

(B) and

0 to 10 mol-% of units of the general formula

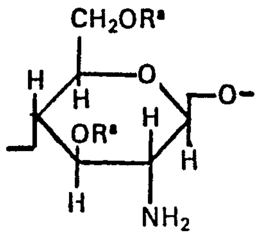

(C)

in a random distribution, wherein $R^a$ means H or $C_1$–$C_6$-alkyl, $R^b$ is H or a residue

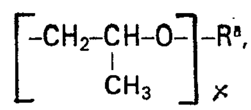

where x = an integer from 1 to 5 and $R^c$ means

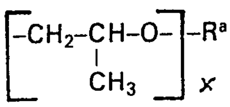

on condition that at least one of the substituents $R^a$ means $C_1$–$C_6$-alkyl in formula A or B.

13. Process for preparing the compound according to Claim 10 to 12, characterised in that a chitosan consisting of 50 to 96% of acetylated chitin, is reacted with propylene oxide in a first reaction step in the presence of an organic dispersion agent at a temperature of 20 °C to 120 °C and the hydroxypropylchitosan obtained is reacted in a second reaction step with an alkyl halide in an alkaline medium at a reaction temperature of 40° to 120 °C, wherein the molecular ration of chitosan to alkylating medium is between 1:3 and 1:15.